(19) Europäisches Patentamt
European Patent Office
Office européen des brevets

(11) Veröffentlichungsnummer: **0 295 408 B1**

## (12) EUROPÄISCHE PATENTSCHRIFT

(45) Veröffentlichungstag der Patentschrift: **11.11.92**

(21) Anmeldenummer: **88107071.8**

(22) Anmeldetag: **03.05.88**

(51) Int. Cl.$^5$: **C12M 1/12**

(54) **Bioreaktor.**

(30) Priorität: **16.06.87 DE 3720049**

(43) Veröffentlichungstag der Anmeldung:
**21.12.88 Patentblatt 88/51**

(45) Bekanntmachung des Hinweises auf die
Patenterteilung:
**11.11.92 Patentblatt 92/46**

(84) Benannte Vertragsstaaten:
**AT BE CH DE ES FR GB IT LI NL SE**

(56) Entgegenhaltungen:
**DE-A- 2 609 825**
**DE-A- 3 539 798**

(73) Patentinhaber: **B. Braun Melsungen AG**
**Carl-Braun Strasse**
**W-3508 Melsungen(DE)**

(72) Erfinder: **Rietschel, Wolfgang**
**Obere Bergstrasse 2**
**W-3501 Söhrewald(DE)**
Erfinder: **Kiel, Reinhard**
**Rhönstrasse 5**
**W-3508 Melsungen(DE)**
Erfinder: **Kahlert, Wolfgang**
**Akazienweg 1**
**W-3501 Körle(DE)**
Erfinder: **Kuhlmann, Winfried, Dr.**
**Schulstrasse 12**
**W-3508 Melsungen(DE)**

(74) Vertreter: **Selting, Günther, Dipl.-Ing. et al**
**Patentanwälte von Kreisler, Selting, Werner**
**Deichmannhaus am Hauptbahnhof**
**W-5000 Köln 1(DE)**

## Beschreibung

Die Erfindung betrifft einen Bioreaktor nach dem Oberbegriff des Patentanspruchs 1 und richtet sich insbesondere auf die Möglichkeit der Dampfsterilisation des Innenraums des Reaktorgefäßes und der unter den Flüssigkeitsspiegel des Gefäßes ragenden Rohre.

Bioreaktoren dienen der Züchtung und dem Wachstum biologischer Substanzen, z.B. von Zellen und Enzymen, und ihr Betrieb erfordert absolute Kontaminationsfreiheit. Bioreaktoren werden aus dampfsterilisierbaren Materialien, z.B. Edelstahl, hergestellt. Zur Beseitigung unerwünschter Keime wird eine Sterilisation mit Dampf durchgeführt, der mit einer Temperatur von 121 ° C im Gefäß gebildet wird oder unter Druck in das Gefäß eingeführt wird. Der Dampf muß nicht nur den Innenraum des Gefäßes sterilisieren, sondern auch das Innere der im Gefäß befindlichen Rohrleitungen, insbesondere der Tauchrohre. Die Sterilisierung eines leeren Bioreaktors ist verhältnismäßig einfach, Schwierigkeiten treten jedoch auf, wenn sich im Bioreaktor eine Flüssigkeit, z.B. Nährflüssigkeit, befindet, weil dann durch den Dampfdruck die Flüssigkeit durch die zu sterilisierenden Rohre hindurch ausgetrieben wird bzw. bei geschlossenen Rohren der Luftraum innerhalb der Rohre nicht mit Dampf sterilisiert wird.

Es ist bekannt, zur Dampfsterilisierung eines Bioreaktors am Gefäß einen separaten Dampfanschluß vorzusehen. Nachteilig ist hierbei, daß das Gefäß aufwendiger wird, und daß der Dampf in die im Gefäß befindliche Flüssigkeit eingeleitet wird. Das Einleiten von Dampf in die Flüssigkeit ist häufig unerwünscht.

Ferner ist es bekannt, in der Wand des Kopfraumes des Gefäßes eine Membran vorzusehen, die zum Einleiten von Dampf mit einer hohlen Anstechnadel durchstochen wird. Hierbei muß die Anstechnadel zuvor mit einer Flamme sterilisiert werden, wobei dennoch Kontaminationen durch die in das Gefäßinnere eingeführte Nadel nicht ganz auszuschließen sind. Die anzuschließenden Leitungen, Filter und anderen Einrichtungen müssen zuvor separat sterilisiert werden.

Aus DE-OS 35 39 798 ist ein Ventil zur Sterilisierung eines Bioreaktors bekannt, wobei das Ventilgehäuse in den Deckel des Bioreaktors eingesetzt ist. Ein Teil des Ventilgehäuses ragt in den Kopfraum des Reaktors hinein und weist dort Öffnungen auf. In einer Bohrung des Ventilgehäuses ist ein Schieber verschiebbar, dessen äußeres Ende an eine Dampfleitung angeschlossen werden kann. Das innere Ende des Schiebers weist zwei in axialem Abstand angeordnete Dichtungen auf. In der Arbeitsstellung des Ventils, bei abgesenktem Schieber, ist der Kanal des Schiebers mit einem sich bis zum Boden des Gefäßes erstreckenden Rohr verbunden und in der Sterilisationsstellung, also bei angehobenem Schieber, gelangt der durch den Kanal des Schiebers zugeführte Dampf durch die dann freiliegende Öffnung des Ventilgehäuses in den Kopfraum des Gefäßes. Dieses Ventil hat den Nachteil, daß in der Sterilisationsstellung der zwischen den beiden Dichtungen befindliche Abschnitt des Schiebers nicht vom Dampf erreicht und nicht sterilisiert wird. Dieser Abschnitt gelangt in der Arbeitsstellung über die Öffnungen in Verbindung mit dem Sterilbereich des Gefäßes, wodurch Kontaminationen des Gefäßinhalts erfolgen können.

Der Erfindung liegt die Aufgabe zugrunde, einen Bioreaktor der im Oberbegriff des Patentanspruchs 1 angegebenen Art zu schaffen, bei dem während der Sterilisation alle Bereiche, die in der Arbeitsphase mit dem Sterilbereich des Gefäßes in Verbindung kommen, sterilisiert werden, so daß Kontaminationen sicher verhindert werden.

Die Lösung dieser Aufgabe erfolgt erfindungsgemäß mit den im kennzeichnenden Teil des Patentanspruchs 1 angegebenen Merkmalen.

Der wesentliche Vorteil des erfindungsgemäßen Bioreaktors besteht darin, daß in der Sterilisationsphase der untere Endbereich des Schiebers durch die Erweiterung der Bohrung des Ventilgehäuses mit Dampf umströmt und somit sterilisiert wird. Der Dampf gelangt auch über eine gewisse Länge in die Bohrung hinein und sterilisiert den unteren Bereich der Bohrungswand. Damit sind alle Teile, die in der Arbeitsphase mit der Öffnung des Ventilgehäuses und mit dem Sterilbereich des Gefäßes in Verbindung kommen können sterilisierbar.

Auch die an das Ventil angeschlossene Leitung und das an diese Leitung angeschlossene Zubehör werden mitsterilisiert und bedürfen keiner separaten Sterilisation.

Vorteilhafte Ausgestaltungen und Weiterbildungen der Erfindung sind den Unteransprüchen zu entnehmen.

Im folgenden wird unter Bezugnahme auf die Zeichnungen ein Ausführungsbeispiel der Erfindung näher erläutert.

Es zeigen:

Fig. 1     eine schematische Darstellung eines Bioreaktors mit den angeschlossenen Leitungen,

Fig. 2     einen Längsschnitt durch das Ventil entlang der Linie II-II von Fig. 1, in der ersten Ventilstellung, also im Zustand "Sterilisation" und

Fig. 3     in gleicher Darstellung wie Fig. 2 die zweite Ventilstellung, die die Arbeitsstellung bildet.

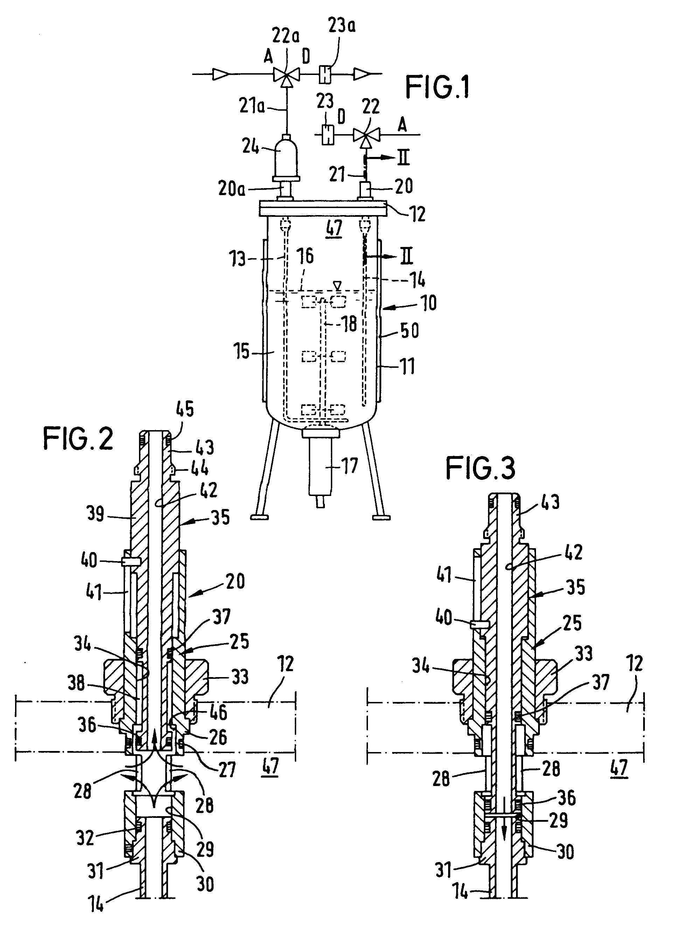

Gemäß Fig. 1 weist der Bioreaktor 10 ein aus Edelstahl bestehendes Gefäß 11 auf, das hermetisch abgedichtet ist. Das Gefäß 11 weist eine Heizvorrichtung 50 auf, die im vorliegenden Fall außen angebracht ist, jedoch auch im Gefäßinneren untergebracht sein könnte. Das Gefäß 11 ist mit einem abdichtend aufgesetzten Deckel 12 verschlossen. Vom Deckel 12 erstrecken sich Rohre 13,14 als Tauchrohre nach unten. Das Rohr 13 ist ein Begasungsrohr, durch das Gas von unten in die im Gefäß 11 befindliche Flüssigkeit 15, deren Pegel mit 16 bezeichnet ist, eingeleitet werden kann. Das Rohr 14 ist ein Zuführungs- bzw. Entnahmerohr zum Zuführen oder Entnehmen von Flüssigkeit. Weitere am Deckel 12 vorgesehene Anschlüsse, z.B. ein Druckanschluß, sowie Durchlässe für Meßsonden u. dgl. sind aus Gründen der Übersichtlichkeit fortgelassen. Unter dem Gefäßboden befindet sich ein Rührwerksantrieb 17 zum Antreiben eines im unteren Gefäßbereichs angeordneten Rührwerks 18.

Das Rohr 14 ist im Innern des Gefäßes 11 mit dem dampfsterilisierbaren Ventil 20 verbunden, das am Deckel 12 befestigt ist und abdichtend durch diesen Deckel hindurchführt. An das Ventil 20 ist außerhalb des Deckels eine Leitung 21 angeschlossen, welche mit dem 3/2-Wegeventil 22 verbunden ist. Das 3/2-Wegeventil 22 weist zwei weitere Anschlüsse D und A auf, wobei der Anschluß D über eine druckreduzierende Blende 23 mit einem Ablauf verbunden ist, während der Arbeitsanschluß A zu einer Zuführungs- oder Entnahmeleitung führt.

Gemäß Fig. 1 ist auch das Rohr 13 mit einem Ventil 20a, das dem Ventil 20 entspricht, verbunden, und dieses Ventil 20a ist über ein Filter 24 und eine Leitung 21a mit dem 3/2-Wegeventil 22a verbunden. Der Anschluß D des 3/2-Wegeventils 22a ist über eine druckreduzierende Blende 23a an einen Ablauf angeschlossen, während der Arbeitsanschluß A mit einer Begasungsleitung verbunden ist.

Normalerweise ist für die Sterilisierung des Bioreaktors nur ein Ventil 20 oder 20a an einem der in die Flüssigkeit eintauchenden Rohre 13 oder 14 erforderlich, jedoch sind zur Verdeutlichung in Fig. 1 beide Rohre mit einem derartigen Ventil versehen. Tatsächlich kann es zweckmäßig sein, jedes der Rohre 13 und 14 mit einem Ventil 20a bzw. 20 auszustatten.

Die nachfolgende Beschreibung des Ventils erfolgt anhand des Ventils 20. Das Ventil 20a ist exakt in gleicher Weise ausgebildet.

Das Ventil 20 weist ein langgestrecktes rohrförmiges Ventilgehäuse 25 auf, das von außen in einen Sitz des Deckels 12 eingesetzt ist und sich mit einem Kragen 26 an einer Ringschulter 12 im Deckeldurchgang abstützt. Unterhalb des Kragens 26 ist in einer Ringnut des Ventilgehäuses 25 eine Dichtung 27 angeordnet, die die Abdichtung zwischen Ventilgehäuse 25 und Deckel 12 bewirkt.

Dicht unterhalb des Deckels 12 weist das Ventilgehäuse 25 seitliche Öffnungen 28 auf und unter diesen Öffnungen befindet sich ein zylindrischer Sitz 29. Unterhalb des Sitzes 29 ist der Rohranschluß 30 mit Gewinde angeordnet, an dem der Kopf 31 des Rohres 14 befestigt ist. Der Kopf 31 ist mit einem Dichtring 32 zum Sitz 29 abgedichtet, so daß der von dem Sitz 29 umschlossene Raum abdichtend mit dem Innern des Rohres 14 in Verbindung steht.

Das Ventilgehäuse 25 ist mit einer von außen gegen den Deckel 12 gesetzten Mutter 33 am Deckel 12 befestigt. Diese Mutter umschließt den zylindrischen Körper des Ventilgehäuses 25 und ist mit einem Außengewinde in ein Innengewinde des Deckeldurchgangs eingeschraubt. Dabei drückt die Mutter den Kragen 26 gegen die Ringschulter des Deckels 12.

In einer vertikalen Längsbohrung 34 des Ventilgehäuses 25 ist der Schieber 35 längsverschiebbar angeordnet. Dieser Schieber 35 hat im wesentlichen zylindrische Form. In der Nähe seines unteren Endes befindet sich die erste Dichtung 36 und mit Abstand darüber befindet sich die zweite Dichtung 37. Zwischen beiden Dichtungen 36 und 37 ist eine Ringnut 38 vorgesehen. Das obere Ende 39 des Schiebers 35 hat einen größeren Durchmesser als derjenige Bereich, in dem sich die Dichtungen 36 und 37 befinden. Die Bohrung des Ventilgehäuses 25 ist auf der Länge, in die der dickere Bereich 39 eintaucht, entsprechend vergrößert. Am unteren Ende des dickeren Bereichs 39 befindet sich ein radial abstehender Führungsstift 40, der in eine Längsnut 41 des Ventilgehäuses 25 hineinragt, um den Schieber 35 gegen Verdrehung relativ zum Ventilgehäuse 25 zu sichern und um den axialen Verschiebebereich des Schiebers 35 zu begrenzen.

Durch den Schieber 35 geht eine axiale Bohrung 42 hindurch, die sich über die gesamte Länge des Schiebers erstreckt. Am oberen Ende des Schiebers 35 befindet sich ein Anschlußkopf 43 mit Gewinde 44 und Dichtung 45 zum Anschluß eines Rohres oder einer anderen Einrichtung, wie z.B. des Filters 24 in Fig. 1.

In der in Fig. 2 dargestellten oberen Stellung des Schiebers 35 befindet sich die erste Dichtung 36 am unteren Ende dieses Schiebers im Bereich einer Erweiterung 46 der Bohrung 34, so daß die Ringnut 38 über die Erweiterung 46 und die Öffnungen 28 mit dem Kopfraum 47 des Gefäßes 11 in Verbindung steht.

Fig. 2 zeigt den Zustand des Ventils 20 in der Stellung "Sterilisation". In dieser Stellung nimmt der Schieber 35 seine obere Endlage ein. Die zweite Dichtung 37 wirkt in jeder der beiden Stellungen des Schiebers 35 mit der Bohrung 34 zusammen und bewirkt in jeder Stellung (und in den Zwischenstellungen) die Abdichtung zwischen Schieber 35 und Ventilgehäuse 25. In der Sterilisationsstellung des Ventils 20 ist das 3/2-Wegeventil 22 auf die Stellung D geschaltet. Die Heizvorrichung heizt die Flüssigkeit bis zur Dampfbildung auf. Der Dampf gelangt aus dem Kopfraum 47 durch die Öffnungen 28 in den Kanal 42 des Schiebers 35. Auf diese Weise werden sämtliche an die Leitung 21 angeschlossenen Teile, einschließlich des Wegeventils 22, des Ventils 20 und des Rohres 14 durch den Dampf sterilisiert. Sterilisiert wird ferner der unterhalb der zweiten Dichtung 37 befindliche Teil der Bohrung 34 und der unterhalb der Dichtung 37 liegende Bereich des Schiebers 35.

In der Arbeitsstellung, die in Fig. 3 dargestellt ist, ist der Schieber 35 in die untere Position geschoben, in der sein die erste Dichtung 36 enthaltendes Ende in den Sitz 29 unterhalb der Öffnungen 28 passend hineinragt. Der Sitz 29 hat den gleichen Durchmesser wie die Bohrung 34. Der Kanal 42 ist nun mit dem Innern des Rohres 14 verbunden, wobei dieser Durchgang gegen den Kopfraum 47 abgedichtet ist. Die Abdichtung des Kopfraums 47 gegen die Umgebung erfolgt auch in dieser Ventilstellung durch die zweite Dichtung 37 des Schiebers 35. In der Arbeitsstellung des Ventils 20 ist das 3/2-Wegeventil 22 auf den Arbeitsanschluß A umgeschaltet, so daß über diesen Arbeitsanschluß die Zufuhr von Medien in das Innere des Gefäßes oder die Entnahme von Gefäßinhalt möglich ist.

Bei dem Ventil 20a, das an das Begasungsrohr 13 angeschlossen ist, ist an dem Kopf 43 des Schiebers 35 das Filter 24a befestigt, das mit der Leitung 21a verbunden ist. Dieses Filter 24 dient gleichzeitig als Griff zum Bewegen des Schiebers 35.

In der Leitung 21 bzw. 21a kann eine Einrichtung zum Längenausgleich vorgesehen sein, um den Schieber in jeder Stellung abdichtend mit dem 3/2-Wegeventil 22 bzw. 22a zu verbinden.

**Patentansprüche**

1. Bioreaktor mit
   - einem abgedichteten Gefäß (11),
   - einem durch eine Außenwand des Gefäßes hindurchgehenden Ventil mit einem Ventilgehäuse (25), das einen in einer Bohrung (34) verschiebbaren Schieber (35) mit einem in Längsrichtung durchgehenden Kanal (42) enthält,
   - wobei am Ventilgehäuse (25) im Kopfraum (47) des Gefäßes (11) eine durch den Schieber (35) verschließbare Öffnung (28) vorgesehen ist und das Ventilgehäuse (25) unterhalb der Öffnung (28) einen Sitz (29) für den Schieber (35) aufweist,
   **dadurch gekennzeichnet,**

daß am unteren Ende der Bohrung (34) eine Erweiterung (46) vorgesehen ist, durch die bei angehobenem Schieber (35) der untere Teil der Bohrung (34) mit dem Kanal (42) in Verbindung steht.

2. Bioreaktor nach Anspruch 1, dadurch gekennzeichnet, daß der Schieber (35) zwei Dichtungen (36, 37) aufweist, zwischen denen eine Ringnut (38) angeordnet ist, wobei die untere Dichtung (36) bei abgesenktem Schieber (35) in den Sitz (29) eingreift und bei angehobenem Schieber im Bereich der Erweitung (46) angeordnet ist, während die obere Dichtung (37) stets in der Bohrung (34) verbleibt.

3. Bioreaktor nach Anspruch 1 oder 2, dadurch gekennzeichnet, daß der Schieber (35) in seiner unteren und seiner oberen Stellung arretierbar ist.

4. Bioreaktor nach einem der Ansprüche 1 bis 3, dadurch gekennzeichnet, daß außerhalb des Gefäßes (11) ein Wegeventil (22) vorgesehen ist, das einen Kondensatauslaß (D) und einen Arbeitsanschluß (A) aufweist, und über eine sterilisierbare Leitung (21) mit dem Schieber (35) des Ventils (20) verbunden ist.

5. Bioreaktor nach Anspruch 4, dadurch gekennzeichnet, daß in der Leitung (21a) zwischen Kondensatanschluß (D) und Arbeitsanschluß (A) einerseits und dem Ventil (20a) andererseits ein Filter (24) angeordnet ist.

6. Bioreaktor nach Anspruch 5, dadurch gekennzeichnet, daß der Auslaß des Filters (24) mit dem Schieber (35) verbunden ist, derart, daß das Filter (24) als Griff zum Bewegen des Schiebers (35) benutzbar ist.

7. Bioreaktor nach einem der Ansprüche 4 bis 6, dadurch gekennzeichnet, daß das Ventil (20) an einem abnehmbaren Deckel (12) des Gefäßes (11) angebracht ist.

8. Bioreaktor nach einem der Ansprüche 4 bis 7, dadurch gekennzeichnet, daß das Ventil (20) automatisch in Verbindung mit der Sterilisationsablaufsteuerung betätigt wird.

**Claims**

1. Bioreactor comprising
   - a sealed vessel (11),
   - a valve extending through an outer wall of the vessel and having a valve chamber (25) accomodating a slide (35) displaceable in a bore (34) and having a channel (42) extending therethrough in the longitudinal direction,

- an opening (28) closable by said slide (35) being provided at the valve chamber (25) in the head space (47) of the vessel (11), and the valve chamber (25) including a seat (29) for the slide (35) beneath the opening (28),

**characterized in**

that at the lower end of the bore (34), there is an enlargement (46) through which, if the slide (35) is lifted, the lower portion of bore (34) communicates with the channel (42).

2. Bioreactor according to claim 1, characterized in that the slide (35) comprises two seals (36, 37) with an annular groove (38) therebetween, the lower seal (36) engaging said seat (29) if the slide (35) is lowered, and being positioned in the area of said enlargement (46), if the slide is lifted, while the upper seal (37) always remains in the bore (34).

3. Bioreactor according to claim 1 or 2, characterized in that the slide may be arrested in its lower and upper positions.

4. Bioreactor according to one of claims 1 to 3, characterized in that outside the vessel (11), a multiway-valve (22) is provided which has a condensate outlet (D) and an operating connection (A) and which is connected through a sterilizable duct (21) to slide (35) of valve (20).

5. Bioreactor according to claim 4, characterized in that in the duct (21a) between condensate connection (D) and operating connection (A), on the one hand, and the valve (20a), on the other hand, a filter (24) is mounted.

6. Bioreactor according to claim 5, characterized in that the outlet of filter (24) is connected to the slide (35) so that the filter (24) may be used as a grip for moving the slide (35).

7. Bioreactor according to one of claims 4 to 6, characterized in that the valve (20) is mounted at a removable cover (12) of the vessel (11).

8. Bioreactor according to one of claims 4 to 7, characterized in that the valve (20) is operated automatically in connection with the control of the sterilizing process.

**Revendications**

1. Bioréacteur comprenant

- un récipient étanche (11),
- une vanne traversant une paroi extérieure du récipient, comportant un boîtier de vanne (25), qui contient un coulisseau (35) susceptible de coulisser dans un alésage (34), et traversé par un canal (42) dans la direction longitudinale,
- une ouverture susceptible d'être fermée par le coulisseau (35) étant prévue sur le boîtier de vanne (25) dans l'espace de tête (47) du récipient (11), et le boîtier de vanne (25) présentant au-dessous de l'ouverture (28) un siège (29) pour la coulisseau (35),

**caractérisé en ce qu'à**

l'extrémité inférieure de l'alésage (34) est prévu un élargissement (46) à travers lequel la partie inférieure de l'alésage (34) est en liaison avec le canal (42) lorsque le coulisseau (35) est soulevé.

2. Bioréacteur selon la revendication 1, caractérisé en ce que le coulisseau (35) présente deux joints d'étanchéité (36,37) entre lesquels est disposée une rainure annulaire (38), le joint inférieur (36) pénétrant dans le siège (29) lorsque le coulisseau (35) est abaissé et étant disposé dans la zone de l'élargissement (46) lorsque le coulisseau est soulevé, tandis que le joint supérieur (37) reste constamment dans l'alésage (34).

3. Bioréacteur selon la revendication 1 ou 2, caractérisé en ce que le coulisseau (35) peut être arrêté dans sa position inférieure et dans sa position supérieure.

4. Bioréacteur selon l'une des revendications 1 à 3, caractérisé en ce qu'il est prévu en dehors du récipient (11) un distributeur (22) qui présente une sortie de condensat (D) et un raccord de travail (A) et est relié par une conduite stérilisable (21) au coulisseau (35) de la vanne (20).

5. Bioréacteur selon la revendication 4, caractérisé en ce qu'un filtre (24) est disposé dans la conduite (21a) entre le raccord de condensat (D) et le raccord de travail (A) d'une part et le vanne (20a) d'autre part.

6. Bioréacteur selon la revendication 5, caractérisé en ce que la sortie du filtre (24) est reliée au coulisseau (35) d'une manière telle que le filtre (24) est utilisable comme saisie pour déplacer le coulisseau (35).

7. Bioréacteur selon l'une des revendications 4 à 6, caractérisé en ce que la vanne (20) est montée sur un couvercle amovible (12) du récipient (11).

8. Bioréacteur selon l'une des revendications 4 à 7, caractérisé en ce que la vanne (20) est automatiquement actionnée en liaison avec la commande d'écoulement de stérilisation.

# FIG.1

# FIG.2

# FIG.3